# EUROPÄISCHE PATENTSCHRIFT

(11) **EP 1 631 207 B1**
(45) Veröffentlichungstag und Bekanntmachung des Hinweises auf die Patenterteilung: **30.08.2006**
(21) Anmeldenummer: 04739504.1
(22) Anmeldetag: 02.06.2004
(51) Int. Cl.: A61B 17/82

(54) **STERNUMVERSCHLUSS**
STERNUM CLOSURE
DISPOSITIF DE FERMETURE DE STERNUM

(30) Priorität: 04.06.2003 DE 10326690
(43) Veröffentlichungstag der Anmeldung: 08.03.2006
(73) Patentinhaber: Aesculap AG & Co. KG, 78532 Tuttlingen (DE)
(72) Erfinder: MORALES, Pedro, 78532 Tuttlingen (DE); DWORSCHAK, Manfred, 78589 Dürbheim (DE); EIJSMAN, Leon, NL-1091 HA Amsterdam (NL)
(74) Vertreter: Boehme, Ulrich
(86) Internationale Anmeldenummer: PCT/EP2004/005918
(87) Internationale Veröffentlichungsnummer: WO 2004/107998

(56) Entgegenhaltungen:
- EP-A- 0 014 823
- US-A- 4 279 248
- US-A- 4 802 477
- US-A- 6 022 351
- US-A- 6 045 552
- PATENT ABSTRACTS OF JAPAN Bd. 2003, Nr. 06, 3. Juni 2003 (2003-06-03) & JP 2003 038504 A (MANII KK), 12. Februar 2003 (2003-02-12)

## Beschreibung

Die Erfindung betrifft einen Sternumverschluß zur Festlegung von zwei miteinander zu verbindenden Sternumteilen mit einem ersten plattenförmigen Anlageelement zur Anlage an der Innenfläche des Sternums, mit mindestens einem daran festgelegten, quer von diesem abstehenden Spannelement, mit einem zweiten plattenförmigen Anlageelement zur Anlage an der Außenseite des Sternums, welches mittels des durch den Zwischenraum zwischen den Sternumteilen hindurchgeführten Spannelements gegen das erste Anlageelement spannbar ist, und mit in Richtung auf das zweite Anlageelement weisenden Fixiervorsprüngen am ersten Anlageelement.

Ein solcher Sternumverschluß ist beispielsweise in der US Patentschrift 4 279 248 beschrieben. Er dient dazu, die zwei Hälften des Sternums nach einer Durchtrennung im Zuge einer Operation in der zusammengeführten Stellung relativ zueinander zu fixieren, so daß die beiden Hälften in der gewünschten Weise zusammenwachsen können. Bei dem bekannten Sternumverschluß sind die plattenförmigen Anlageelemente mit seitlichen spitzen, zahnförmigen Vorsprüngen versehen, die sich beim Gegeneinanderspannen der beiden Anlageelemente in das Sternum eingraben und dadurch die Sternumteile relativ zueinander sicher festlegen. Allerdings erfolgt das Eingraben dieser Vorsprünge erst beim Gegeneinanderspannen der Anlageelemente. Die Anlage selbst ist recht kompliziert, da Sorge dafür getragen werden muß, daß das erste Anlageelement, das an der Innenseite des,Sternums angelegt werden soll, in seiner Anlageposition bleibt. Dazu ist es bei der bekannten Vorrichtung notwendig, die als Gewindestäbe ausgebildeten Spannelemente an einem speziellen Stützelement festzulegen.

Es ist Aufgabe der Erfindung, einen gattungsgemäßen Sternumverschluß so auszubilden, daß die Anlage des Sternumverschlusses erleichtert wird.

Diese Aufgabe wird bei einem Sternumverschluß der eingangs beschriebenen Art erfindungsgemäß dadurch gelöst, daß mindestens ein Fixiervorsprung an dem ersten Anlageelement mit widerhakenförmigen Rücksprüngen versehen ist. Auf diese Weise ist es möglich, das erste Anlageelement vor dem Zusammenspannen der Sternumteile an der Innenseite eines der beiden Sternumteile so sicher festzulegen, daß das Anlageelement bis zum Verspannen an diesem Sternumteil sicher gehalten wird. Die Halterung erfolgt dabei dadurch, daß sich ein Vorsprung mit Widerhaken beim Andrücken des ersten Anlageelementes an die Innenseite des Sternumteiles in dieses eingräbt und durch die Widerhaken das Anlageelement an der Innenseite des Sternumteiles so sicher festhält, daß der Operateur keine weiteren Hilfsmittel braucht, um das Anlageelement bis zum Zusammenführen der Sternumteile und dem anschließenden Zusammenspannen der Anlageelemente in dieser Position zu lassen.

Dabei ist es vorteilhaft, wenn mindestens ein mit Rücksprüngen versehener Fixiervorsprung weiter in Richtung auf das zweite Anlageelement vorsteht als andere Fixiervorsprünge. Mit anderen Worten, es sind die Fixiervorsprünge mit widerhakenförmigen Rücksprüngen vorzugsweise länger ausgebildet als andere Fixiervorsprünge, so daß diese tiefer in das Sternumgewebe eindringen, wenn das erste Anlageelement gegen das Sternum gedrückt wird.

Die widerhakenförmigen Rücksprünge können dabei einfache, sägezahnförmige Rücksprünge sein, es kann sich um Mikrohaken handeln, d. h. die Oberfläche oder die Seitenkanten der Vorsprünge werden mit kleinen Stufen versehen, es kann sich aber auch um relativ große Rücksprünge handeln, wesentlich ist nur, daß sich die mit widerhakenförmigen Rücksprüngen versehenen Fixiervorsprünge zwar leicht in das Gewebe des Sternums eindrücken lassen, jedoch sehr schwer wieder herauszuziehen sind.

Bei einer ersten bevorzugten Ausführungsform ist vorgesehen, daß das erste Anlageelement nur einen Fixiervorsprung mit Rücksprüngen und eine Vielzahl von Fixiervorsprüngen ohne Rücksprünge aufweist.

Es ist günstig, wenn die Fixiervorsprünge an im wesentlichen parallel zu dem Zwischenraum zwischen den Sternumteilen verlaufenden Enden relativ zu einer senkrecht zur Anlagefläche des Anlageelements liegenden Ebene geringfügig geneigt sind. Durch diese Neigung können die Fixiervorsprünge die Sternumteile gegeneinander spannen, wenn die Anlageelemente gegeneinander gespannt werden.

Dazu ist es beispielsweise vorteilhaft, daß die geneigten Fixiervorsprünge auf gegenüberliegenden Seiten aufeinander zugeneigt sind.

Grundsätzlich können die Anlageelemente über ein einziges Spannelement gegeneinander gespannt werden, es ist aber vorteilhaft, wenn an dem ersten Anlageelement zwei Spannelemente im Abstand und parallel zueinander verlaufend festgelegt sind.

Das Spannelement kann stiftförmig ausgebildet sein. Es sind aber auch bandförmige oder stabförmige Ausgestaltungen möglich.

Es ist vorteilhaft, wenn das zweite Anlageelement mindestens eine elastische Zunge trägt, die an dem Spannelement anlegt und eine Verschiebung des Anlageelementes gegenüber dem Spannelement nur in einer Richtung ermöglicht. Dadurch kann das zweite Anlageelement längs des Spannelementes ohne weiteres gegen das erste Anlageelement verschoben werden, wird aber dann durch die elastische Zunge oder die elastischen Zungen daran gehindert, sich wieder von dem ersten Anlageelement zu entfernen. Beispielsweise können diese Zungen durch radiale Schlitze gebildet werden, die von einer Öffnung im zweiten Anlageelement ausgehen, durch welche das Spannelement hindurchtritt.

Bei einer abgewandelten Ausführungsform ist vorgesehen, daß auf der dem ersten Anlageelement abgewandten Seite des zweiten Anlageelementes ein auf dem Spannelement verschiebbares Halteglied angeordnet ist, welches mindestens eine elastische Zunge trägt, die an dem Spannelement anliegt und eine Verschiebung des Haltegliedes gegenüber dem Spannelement nur in einer Richtung ermöglicht. In diesem Falle ist also die elastische Zunge nicht im Anlageelement selbst angeordnet, sondern in einem separaten Halteglied, das auf dem Spannelement verschiebbar ist und dadurch das zweite Anlageelement gegen das erste Anlageelement spannt.

Es ist gemäß einer bevorzugten Ausführungsform vorgesehen, daß die Anlageelemente rechteckig ausgebildet sind.

Vorzugsweise ist dabei ein Fixiervorsprung mit widerhakenförmigen Rücksprüngen in der Mitte der längeren Seitenkante des ersten Anlageelementes angeordnet.

Bei einer bevorzugten Ausführungsform sind die Fixiervorsprünge am zweiten Anlageelement gleich ausgebildet wie die Fixiervorsprünge am ersten Anlageelement. In diesem Falle trägt also auch das zweite Anlageelement mindestens einen Fixiervorsprung mit widerhakenförmigen Rücksprüngen, vorzugsweise einen Fixiervorsprung, der länger ausgebildet ist als Fixiervorsprünge ohne derartige Rücksprünge.

Die nachfolgende Beschreibung bevorzugter Ausführungsformen der Erfindung dient im Zusammenhang mit der Zeichnung der näheren Erläuterung. Es zeigen:
- Figur 1:: eine schematische Vorderansicht von zwei Sternumhälften mit drei längs der Trennlinie zwischen den beiden Sternumhälften angeordneten Sternumverschlüssen;
- Figur 2:: eine perspektivische Ansicht eines Sternumverschlusses vor dem Gegeneinanderspannen der Anlageelemente;
- Figur 3:: eine Längsschnittansicht durch einen Sternumverschluß nach der Anlage an den beiden Sternumhälften und im gespannten Zustand und
- Figur 4:: eine Ansicht des Sternumverschlusses der Figur 3 in Richtung des Pfeiles A in Figur 3.

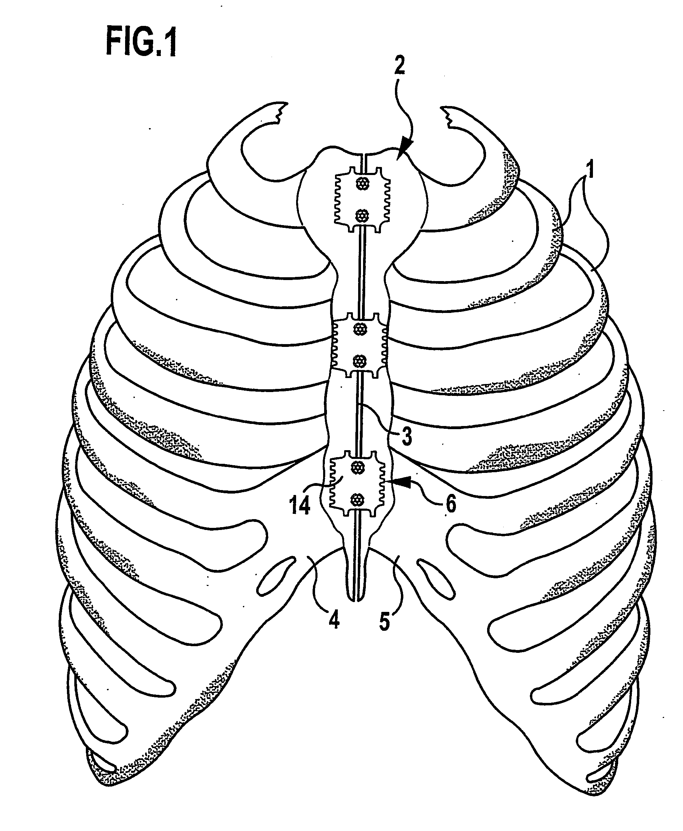

In Figur 1 sind die Rippenbögen 1 eines Patienten dargestellt, längs des Sternums 2 sind diese durch einen Trennschnitt 3 geteilt, um dadurch den Zugang zum Innenraum des Brustkorbes zu eröffnen. Nach erfolgter Operation muß längs des Trennschnittes 3 eine Verbindung der beiden Sternumteile 4, 5 erfolgen, und dazu werden die beiden Sternumteile 4, 5 mittels eines in der Zeichnung nicht dargestellten Werkzeuges, beispielsweise mittels einer Spannzange, gegeneinander gedrückt und müssen in dieser Lage relativ zueinander fixiert werden.

Diese Fixierung erfolgt über im Abstand zueinander angeordnete Sternumverschlüsse 6, im dargestellten Ausführungsbeispiel werden drei derartige Sternumverschlüsse verwendet.

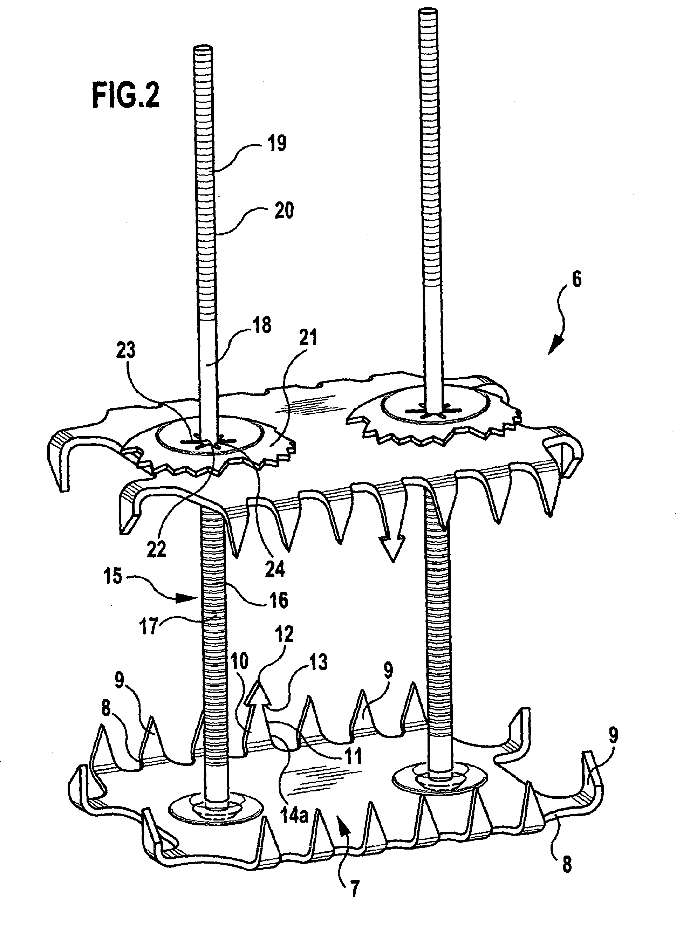

Jeder Sternumverschluß 6 ist gleich aufgebaut, anhand der Figuren 2 bis 4 wird nachstehend dieser Aufbau erläutert.

Der Sternumverschluß 6 umfaßt ein rechteckiges, plattenförmiges erstes Anlageelement 7 mit in allen Richtungen von ihm abstehenden Armen 8, die an ihren freien Enden im wesentlichen senkrecht abgebogen sind und dort seitlich abgeschrägte zahnförmige Fixiervorsprünge 9 ausbilden. Die meisten Fixiervorsprünge werden zur Spitze hin kontinuierlich schmaler, lediglich ein einzelner Fixiervorsprung 10, der in der Mitte der Längsseite des Anlageelementes 7 angeordnet und länger ausgebildet ist als die übrigen Fixiervorsprünge 9, weist einen Querschnitt auf, der etwa Pfeilform hat, so daß dadurch auf gegenüberliegenden Seiten zwei Rücksprünge 11 ausgebildet werden. Diese Rücksprünge werden an ihrem der Spitze 12 zugewandten Ende durch eine quer zur Längsrichtung des Fixiervorsprunges 10 verlaufende Kante 13 begrenzt, im übrigen durch eine Kante 14a, die relativ zur Längsrichtung des Fixiervorsprunges 10 schräg verläuft, so daß die Breite des Fixiervorsprunges 10 zur Spitze 12 hin abnimmt. Der Rücksprung 11 bildet somit einen Widerhaken aus, beim Anlegen des Anlageelementes 7 an die Innenseite eines Sternumteils 4 drücken sich alle Fixiervorsprünge 9 und 10 in das Knochen- und Knorpelgewebe ein, dabei verhindert der widerhakenförmige Rücksprung 11 ein Herausziehen des Fixiervorsprunges 10 aus diesem Gewebe und hält somit das Anlageelement 7 sicher an der Innenseite des entsprechenden Sternumteiles.

Ein zweites Anlageelement ist hinsichtlich der beschriebenen Merkmale identisch aufgebaut wie das erste Anlageelement 7, es wird im Sternumverschluß 6 dem ersten Anlageelement 7 gegenüberliegend so angeordnet, daß die Fixiervorsprünge 9 und 10 des ersten Anlageelementes 7 und des zweiten Anlageelementes 14 mit ihren Spitzen gegeneinander gerichtet sind.

Das erste Anlageelement 7 trägt im Abstand zueinander zwei parallel verlaufende Stifte 15, diese sind dauerhaft mit dem ersten Anlageelement 7 verbunden, beispielsweise durch Einschrauben oder durch Vernietung oder Verschweißung. Der Stift 15 ist in dem an das erste Anlageelement 7 anschliessenden Abschnitt 16 mit im Querschnitt sägezahnförmigen Umfangsnuten 17 versehen, daran schließt sich ein glatter Abschnitt 18 an und schließlich ein weiterer mit einer Profilierung 19 versehener Greifabschnitt 20.

Die beiden Stifte 15 treten durch Öffnungen im zweiten Anlageelement 14 hindurch und tragen auf der Außenseite des zweiten Anlageelementes 14 zwei scheibenförmige Halteglieder 21, die ebenfalls eine zentrale Öffnung 22 für den Durchtritt des Stiftes 15 aufweisen. Von dieser zentralen Öffnung 22 gehen radiale Schnitte 23 aus, so daß dreieckförmige Zungen 24 aus dem Material des Haltegliedes 21 herausgeschnitten sind. Diese Zungen sind durch entsprechende Materialwahl der Halteglieder 21 elastisch aus ihrer Ebene abbiegbar und liegen mit ihren Spitzen am Umfang des Stiftes 15 an. Schiebt man das Halteglied 21 auf dem Stift 15 in Richtung auf das erste Anlageelement 7, so biegen sich die Zungen 24 elastisch nach oben und ermöglichen diese Verschiebung, eine Verschiebung in der umgekehrten Richtung wird aber dadurch verhindert, daß die nach oben gebogenen Zungen 24 sich gegenüber dem Umfang des Stiftes 15 verkeilen und in diesen einschneiden und in die Umfangsnuten 17 eingreifen, so daß auf diese Weise nur eine Verschiebung des Haltegliedes 21 in einer Richtung erfolgen kann, die nachfolgend als die Spannrichtung bezeichnet wird. Beim Verschieben der Halteglieder 21 auf den entsprechenden Stiften 15 legen sich diese an die Außenseite des zweiten Anlageelementes 14 an und verschieben dieses in Richtung auf das erste Anlageelement 7, die beiden Anlageelemente 7 und 14 werden dadurch gegeneinander gespannt und durch die Halteglieder 21 in dieser Haltung fixiert. Dabei tauchen die Fixiervorsprünge 9 und 10 in das Gewebe des Sternums 2 ein und legen die Sternumteile 4 und 5 relativ zueinander sicher fest.

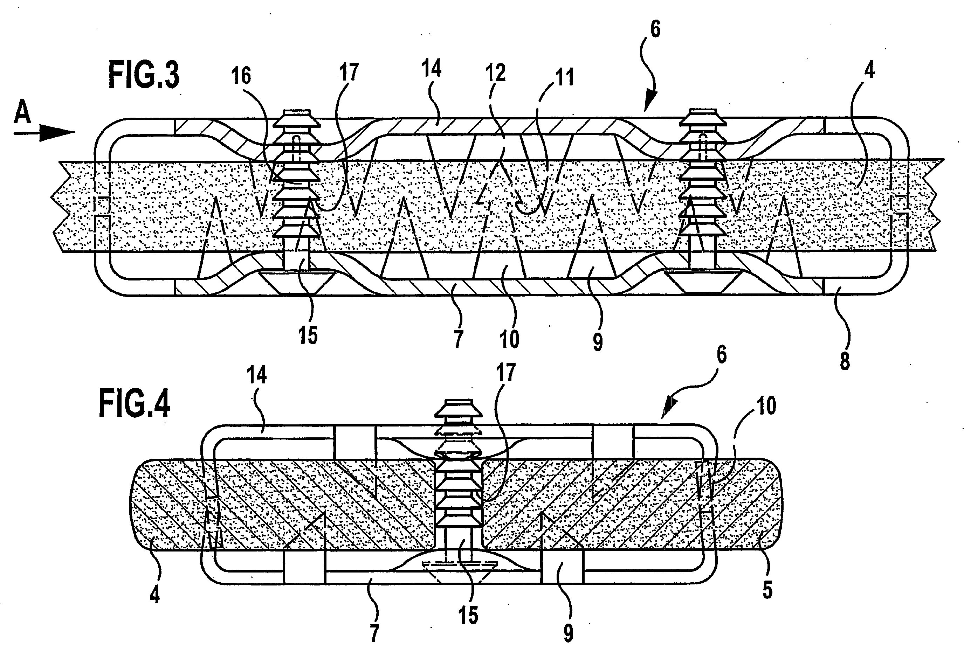

Bei dem Ausführungsbeispiel der Figuren 3 und 4 werden keine Halteglieder 21 verwendet, sondern die Öffnungen im zweiten Anlageelement 14, durch welche die Stifte 15 hindurchtreten, sind so ausgebildet wie die zentrale Öffnung 22 im Halteglied 21, es sind also Schnitte vorgesehen, die elastische Zungen aus dem Material des zweiten Anlageelementes 14 heraustrennen. Auf diese Weise kann das zweite Anlageelement 14 längs der Stifte 15 zwar in die Spannrichtung verschoben werden, nicht aber in die entgegengesetzte Richtung.

Wie aus der Darstellung der Figur 4 zu entnehmen ist, sind einige Fixiervorsprünge 9 gegenüber der Spannrichtung geringfügig geneigt, so daß beim Zusammenspannen der Anlageelemente 7 und 14 eine Querkomponente auf die Sternumteile 4, 5 ausgeübt wird und diese zusätzlich gegeneinander spannt.

Beim Anlegen der Sternumverschlüsse kann der Operateur die ersten Anlageelemente 7 zwischen den Sternumteilen 4, 5, die noch einen gewissen Abstand voneinander einhalten, hindurch in den Innenraum des Körpers einführen und dann an der Innenseite eines Sternumteiles dadurch dauerhaft festlegen, daß der Fixiervorsprung 10 mit dem widerhakenförmigen Rücksprung 11 in das Gewebe des entsprechenden Sternumteils eingedrückt wird. Da dies nur an einem Sternumteil notwendig ist, ist bei dem bevorzugten Ausführungsbeispiel ein entsprechender Fixiervorsprung 10 mit widerhakenförmigen Rücksprüngen nur an einer Seite des Anlageelementes 7 angeordnet.

Dadurch wird nicht nur das erste Anlageelement 7 an dem Sternumteil festgelegt, sondern auch die fest mit ihm verbundenen Stifte 15, die so positioniert werden, daß sie unmittelbar neben dem Trennschnitt 3 angeordnet sind. Die Sternumteile werden jetzt mittels eines geeigneten Werkzeuges gegeneinander gedrückt, und das zweite Anlageelement 14 wird durch ein geeignetes Werkzeug gegen das erste Anlageelement 7 gespannt. Dazu kann beispielsweise ein zangenförmiges Instrument verwendet werden, welches den profilierten Greifabschnitt 20 erfaßt und gleichzeitig mit einem zweiten Teil die Halteglieder 21 oder das zweite Anlageelement 14 in Richtung auf das erste Anlageelement 7 verschiebt.

Nach Beendigung des Spannvorganges greifen auch die Fixiervorsprünge 9, 10 des zweiten Anlageelementes 14 in das Gewebe des Sternums 2 ein, und die beiden Sternumteile 4, 5 sind dauerhaft und sicher relativ zueinander festgelegt. Die überstehenden Teile der Stifte 15 werden entfernt, und die Sternumverschlüsse verbleiben als Implantat im Körper.

Es ist aus diesem Grund auch vorteilhaft, wenn die Sternumverschlüsse aus einem resorbierbaren Material hergestellt werden, so daß sie nach erfolgter Heilung allmählich abgebaut werden können. In anderen Fällen werden aber übliche körperkompatible metallische Werkstoffe verwendet, beispielsweise Titan oder Titanlegierungen.

## Patentansprüche

1. Sternumverschluß (6) zur Festlegung von zwei miteinander zu verbindenden Sternumteilen (4, 5) mit einem ersten plattenförmigen Anlageelement (7) zur Anlage an der Innenfläche des Sternums (2), einem daran festgelegten, quer von diesem abstehenden Spannelement (15), mit einem zweiten plattenförmigen Anlageelement (14) zur Anlage an der Außenseite des Sternums (2), welches mittels des durch den Zwischenraum zwischen den Sternumteilen (4, 5) hindurchgeführten Spannelements (15) gegen das erste Anlageelement (7) spannbar ist, und mit in Richtung auf das zweite Anlageelement (14) weisenden Fixiervorsprüngen (9, 10) am ersten Anlageelement (7), **dadurch gekennzeichnet, daß** mindestens ein Fixiervorsprung (10) am ersten Anlageelement (7) mit widerhakenförmigen Rücksprüngen (11) versehen ist.

2. Sternumverschluß nach Anspruch 1, **dadurch gekennzeichnet, daß** mindestens ein mit Rücksprüngen (11) versehener Fixiervorsprung (10) weiter in Richtung auf das zweite Anlageelement (14) vorsteht als andere Fixiervorsprünge (9).

3. Sternumverschluß nach einem der Ansprüche 1 oder 2, **dadurch gekennzeichnet, daß** das erste Anlageelement (7) nur einen Fixiervorsprung (10) mit Rücksprüngen (11) und eine Vielzahl von Fixiervorsprüngen (9) ohne Rücksprünge aufweist.

4. Sternumverschluß nach einem der voranstehenden Ansprüche, **dadurch gekennzeichnet, daß** mindestens ein Fixiervorsprung (9) relativ zu einer senkrecht zur Anlagefläche des Anlageelementes (7) liegenden Ebene geringfügig geneigt ist.

5. Sternumverschluß nach Anspruch 4, **dadurch gekennzeichnet, daß** die geneigten Fixiervorsprünge (9) auf gegenüberliegenden Seiten aufeinander zugeneigt sind.

6. Sternumverschluß nach einem der voranstehenden Ansprüche, **dadurch gekennzeichnet, daß** an dem ersten Anlageelement (7) zwei Spannelemente (15) im Abstand und parallel zueinander verlaufend festgelegt sind.

7. Sternumverschluß nach einem der voranstehenden Ansprüche, **dadurch gekennzeichnet, daß** das Spannelement (15) stiftförmig ausgebildet ist.

8. Sternumverschluß nach einem der voranstehenden Ansprüche, **dadurch gekennzeichnet, daß** das zweite Anlageelement (14) elastische Zungen (24) trägt, die an dem Spannelement (15) anliegen und eine Verschiebung des Anlageelementes (14) gegenüber dem Spannelement (15) nur in einer Richtung ermöglichen.

9. Sternumverschluß nach einem der Ansprüche 1 bis 7, **dadurch gekennzeichnet, daß** auf der dem ersten Anlageelement (7) abgewandten Seite des zweiten Anlageelementes (14) ein auf dem Spannelement (15) verschiebbares Halteglied (21) angeordnet ist, welches elastische Zungen (24) trägt, die an dem Spannelement (15) anliegen und eine Verschiebung des Haltegliedes (21) gegenüber dem Spannelement (15) nur in einer Richtung ermöglichen.

10. Sternumverschluß nach einem der voranstehenden Ansprüche, **dadurch gekennzeichnet, daß** die Anlageelemente (7, 14) rechteckig ausgebildet sind.

11. Sternumverschluß nach Anspruch 10, **dadurch gekennzeichnet, daß** ein Fixiervorsprung (10) mit widerhakenförmigen Rücksprüngen (11) in der Mitte der längeren Seitenkante des ersten Anlageelementes (7) angeordnet ist.

12. Sternumverschluß nach einem der voranstehenden Ansprüche, **dadurch gekennzeichnet, daß** die Fixiervorsprünge (9, 10) am zweiten Anlageelement (14) gleich ausgebildet sind wie am ersten Anlageelement (7).

## Claims

1. Sternum closure device (6) for fixing two sternum portions (4, 5) to be joined to each other, comprising a first plate-shaped contact element (7) for placement against the inner surface of the sternum (2), a clamping element (15) fixed on and protruding transversely from the first contact element, a second plate-shaped contact element (14) for placement against the outer side of the sternum (2), the second contact element being clampable against the first contact element (7) by means of the clamping element (15) passing through the space between the sternum portions (4,5), and fixing projections (9, 10) on the first contact element (7) pointing in the direction towards the second contact element (14), **characterized in that** at least one fixing projection (10) on the first contact element (7) is provided with barb-shaped recesses (11).

2. Sternum closure device in accordance with claim 1, **characterized in that** at least one fixing projection (10) provided with recesses (11) protrudes further in the direction towards the second contact element (14) than other fixing projections (9).

3. Sternum closure device in accordance with claim 1 or 2, **characterized in that** the first contact element (7) comprises only one fixing projection (10) with recesses (11) and a plurality of fixing projections (9) without recesses.

4. Sternum closure device in accordance with any one of the preceding claims, **characterized in that** at least one fixing projection (9) is slightly inclined relative to a plane lying perpendicular to the contact surface of the contact element (7).

5. Sternum closure device in accordance with claim 4, **characterized in that** the inclined fixing projections (9) are inclined towards one another on opposite sides.

6. Sternum closure device in accordance with any one of the preceding claims, **characterized in that** two clamping elements (15) are fixed on the first contact element (7) so as to extend in parallel spaced relation to each other.

7. Sternum closure device in accordance with any one of the preceding claims, **characterized in that** the clamping element (15) is of pin-shaped design.

8. Sternum closure device in accordance with any one of the preceding claims, **characterized in that** the second contact element (14) carries elastic tongues (24) which rest against the clamping element (15) and enable displacement of the contact element (14) relative to the clamping element (15) in one direction only.

9. Sternum closure device in accordance with any one of claims 1 to 7, **characterized in that** a holding member (21) displaceable on the clamping element (15) is arranged on the side of the second contact element (14) facing away from the first contact element (7) and carries elastic tongues (24) which rest against the clamping element (15) and enable displacement of the holding member (21) relative to the clamping element (15) in one direction only.

10. Sternum closure device in accordance with any one of the preceding claims, **characterized in that** the contact elements (7, 14) are of rectangular design.

11. Sternum closure device in accordance with claim 10, **characterized in that** a fixing projection (10) with barb-shaped recesses (11) is arranged at the centre of the longer side edge of the first contact element (7).

12. Sternum closure device in accordance with any one of the preceding claims, **characterized in that** the fixing projections (9, 10) on the second contact element (14) are of the same design as on the first contact element (7).

## Revendications

1. Dispositif de fermeture de sternum (6) pour la fixation de deux parties de sternum (4, 5) à raccorder comportant un premier élément d'appui en forme de plaque (7) pour appui sur la surface interne du sternum (2), un élément de serrage (15) fixé à celui-ci, éloigné de celui-ci transversalement, comportant un deuxième élément d'appui en forme de plaque (14) pour appui sur la face externe du sternum (2), lequel peut être serré au moyen de l'élément de serrage (15), inséré à travers l'interstice entre les parties de sternum (4, 5), contre le premier élément d'appui (7), et comportant des saillies de fixation (9, 10) sur le premier élément d'appui (7) orientées en direction du second élément d'appui (14), **caractérisé en ce qu'**au moins une saillie de fixation (10) sur le premier élément d'appui (7) est munie de retraits en forme d'ardillons (11).

2. Dispositif de fermeture de sternum selon la revendication 1, **caractérisé en ce qu'**au moins une saillie de fixation (10) munie de retraits (11) dépasse plus loin, en direction du second élément d'appui (14), que d'autres saillies de fixation (9).

3. Dispositif de fermeture de sternum selon l'une quelconque des revendications 1 ou 2, **caractérisé en ce que** le premier élément d'appui (7) ne comporte qu'une saillie de fixation (10) avec des retraits (11) et une multitude de saillies de fixation (9) sans retraits.

4. Dispositif de fermeture de sternum selon l'une quelconque des revendications précédentes, **caractérisé en ce qu'**au moins une saillie de fixation (9) est légèrement inclinée par rapport à un plan se trouvant perpendiculairement à la surface d'appui de l'élément d'appui (7).

5. Dispositif de fermeture de sternum selon la revendication 4, **caractérisé en ce que** les saillies de fixation inclinées (9) sont inclinées les unes en direction des autres sur des côtés opposés.

6. Dispositif de fermeture de sternum selon l'une quelconque des revendications précédentes, **caractérisé en ce que** deux éléments de serrage (15) sont fixés sur le premier élément d'appui (7) à distance et parallèlement l'un par rapport à l'autre.

7. Dispositif de fermeture de sternum selon l'une quelconque des revendications précédentes, **caractérisé en ce que** l'élément de serrage (15) est réalisé sous forme de goupille.

8. Dispositif de fermeture de sternum selon l'une quelconque des revendications précédentes, **caractérisé en ce que** le second élément d'appui (14) comporte des languettes élastiques (24) qui sont en contact avec l'élément de serrage (15) et permettent un déplacement de l'élément d'appui (14) par rapport à l'élément de serrage (15) uniquement dans une direction.

9. Dispositif de fermeture de sternum selon l'une quelconque des revendications 1 à 7, **caractérisé en ce qu'**est disposé, sur le côté du second élément d'appui (14), ne faisant pas face au premier élément d'appui (7), un élément de maintien (21) déplaçable sur l'élément de serrage (15), lequel comporte des languettes élastiques (24) qui sont contact avec l'élément de serrage (15) et permettent un déplacement de l'élément de maintien (21) par rapport à l'élément de serrage (15) uniquement dans une direction.

10. Dispositif de fermeture de sternum selon l'une quelconque des revendications précédentes, **caractérisé en ce que** les éléments d'appui (7, 14) sont réalisés en rectangle.

11. Dispositif de fermeture de sternum selon la revendication 10, **caractérisé en ce qu'**une saillie de fixation (10) avec des retraits en forme d'ardillons (11) est disposée au milieu de l'arête latérale la plus longue du premier élément d'appui (7).

12. Dispositif de fermeture de sternum selon l'une quelconque des revendications précédentes, **caractérisé en ce que** les saillies de fixation (9, 10) sur le second élément d'appui (14) sont réalisées de la même façon que sur le premier élément d'appui (7).
